# FASCICULE DE BREVET EUROPEEN

(11) **EP 3 773 271 B1**
(45) Date de publication et mention de la délivrance du brevet: **11.05.2022**
(21) Numéro de dépôt: 19713491.9
(22) Date de dépôt: 29.03.2019
(51) Int. Cl.: A61B 17/34, A61F 2/24, A61N 1/05

(54) **ENSEMBLE DE POSE D'UN IMPLANT CARDIAQUE, AORTIQUE OU ARTERIEL AVEC ASSISTANCE DE STIMULATION PAR CATHETER POUR VOIE ARTERIELLE OU VEINEUSE PERIPHERIQUE**
ANORDNUNG ZUR EINSETZUNG EINES HERZ-, AORTEN- ODER ARTERIENIMPLANTATS MIT STIMULATIONSUNTERSTÜTZUNG DURCH EINEN PERIPHEREN VENÖSEN ODER ARTERIELLEN KATHETER
ASSEMBLY FOR PLACEMENT OF A CARDIAC, AORTIC OR ARTERIAL IMPLANT WITH STIMULATION ASSISTANCE BY A PERIPHERAL VENOUS OR ARTERIAL CATHETER

(30) Priorité: 29.03.2018 FR 1852770; 02.05.2018 FR 1853783
(43) Date de publication de la demande: 17.02.2021
(73) Titulaire: Electroducer, 38000 Grenoble (FR)
(72) Inventeur: FAURIE, Benjamin, 38240 Meylan (FR)
(74) Mandataire: Nony
(86) Numéro de dépôt international: PCT/EP2019/058038
(87) Numéro de publication internationale: WO 2019/185880

(56) Documents cités:
- FR-A1- 3 034 650
- US-A- 5 733 323
- US-A1- 2009 270 941
- US-A1- 2011 040 344
- US-A1- 2013 096 555

## Description

### Domaine technique

La présente invention concerne un ensemble de pose d'un implant cardiaque, aortique ou artériel.

Par « implant cardiaque ou aortique », on entend ici et dans le cadre de l'invention, une valve aortique ou cardiaque prothétique destinée au remplacement d'une valve native.

Une application privilégiée est le remplacement d'une valve cardiaque par voie percutanée, comprenant un cathéter de délivrance de valve et le cas échéant un dispositif d'introduction, couramment appelé « introducteur ».

Une opération de remplacement d'une valve cardiaque par voie percutanée est connue usuellement sous l'acronyme anglo-saxon TAVI pour « Transcatheter Aortic Valve Replacement ».

Bien que décrit en référence au remplacement d'une valve aortique, l'ensemble selon l'invention peut tout aussi bien s'appliquer pour le remplacement d'une autre valve du cœur, telle que la valve triscupide ou la valve mitrale.

Par «implant artériel», on entend ici et dans le cadre de l'invention, un dispositif prothétique qui peut être un support (ballonnet, stent) destiné à être implanté dans une artère d'un être humain pour réparer (dilater, ré-ouvrir) une artère endommagée.

L'invention s'applique à tous les types d'interventions vasculaires dites périphériques, sur les artères périphériques, comme les artères carotides, les aortes, notamment les opérations de pose d'endoprothèses aortiques thoraciques (TEVAR acronyme anglo-saxon pour « Thoracic Endovascular Aortic Repair »), de pose de stents carotidiens pour les personnes souffrant d'un rétrécissement de ces artères (CAS acronyme anglo-saxon pour « Carotid Artery Stenting »). Il peut s'agir d'interventions cardiaques ou périphériques en pédiatrie, en particulier dans le cas de cardiopathies congénitales.

La présente invention a trait plus particulièrement à l'amélioration de l'assistance à la pose par sidération cardiaque au moyen d'un stimulateur cardiaque afin de stabiliser, de sécuriser et d'optimiser la précision de pose des différents dispositifs comme les stents, ballons ou autres prothèses.

De manière générale, l'introducteur et/ou le cathéter de délivrance de l'ensemble selon l'invention peut être implanté par voie percutanée au sein d'un patient, et plus précisément, par voie trans-fémorale, par voie trans-aortique, par voie carotidienne ou par voie dite sous-clavière, ou dans les artères périphériques comme les artères radiales ou cubitales.

### Etat de la technique

Une maladie cardiaque largement connue est celle liée au rétrécissement par calcification de la valve aortique cardiaque, cette dernière étant la valve qui sépare une cavité cardiaque, à savoir le ventricule gauche de l'aorte et qui permet en position ouverte de laisser passer le sang du cœur vers le reste de l'organisme d'un être humain.

Un rétrécissement serré ou très serré empêche la valve aortique de s'ouvrir normalement et donc génère la maladie aussi appelée sténose aortique calcifiée.

Le traitement de cette maladie consiste à remplacer la valve aortique cardiaque défectueuse.

Le remplacement d'une valve aortique cardiaque défectueuse est le plus fréquemment, réalisé par ouverture du thorax, mise du patient sous circulation extracorporelle, arrêt cardiaque temporaire, et ouverture du coeur, en vue de l'exérèse et du remplacement de la valve native par une valve artificielle ou de prothèse.

Ces étapes successives de l'opération ont pour inconvénients majeurs d'impliquer une hospitalisation relativement longue du patient, d'être complexe et coûteuse et de n'être réservée qu'à une partie des patients atteints, car dans bon nombre de cas, le(s) médecin(s) et/ou chirurgien(s) considèrent que l'intervention chirurgicale dite « à cœur ouvert » ne peut être pratiquée car trop risquée compte tenu de l'état général du patient, notamment du fait de l'arrêt du cœur nécessaire et de la circulation extracorporelle afférente.

Pour remédier à cet inconvénient, il a été envisagé de remplacer une valve cardiaque par voie peu invasive mais toujours sous circulation extracorporelle. On peut citer ici les demandes de brevet internationales WO 93/01768 et WO 97/28807, ainsi que les brevets américains US 5814097, US 5370685 ou US 5545214 qui illustrent des techniques peu invasives connues ainsi que des instruments de mise en œuvre de ces techniques.

Les techniques existantes ont toutefois été considérées comme n'étant pas parfaitement satisfaisantes et comme susceptibles d'être améliorées.

En particulier, ces techniques ont comme inconvénients majeurs :
- d'imposer en tout état de cause la mise du patient sous circulation extracorporelle; elles sont difficiles à mettre en pratique;
- de ne pas permettre un contrôle précis du diamètre selon lequel la valve native est coupée, en vue du calibrage ultérieur de la valve prothétique;
- d'entraîner des risques de diffusion de fragments de valve native, souvent calcifiée, dans l'organisme, qui peuvent conduire à une embolie,
- des risques de perforation de la paroi aortique ou cardiaque ;
- des risques de reflux aigus du sang lors de l'ablation de la valve native.

Pour pallier les insuffisances de ces techniques, une approche a été la mise en place de valves aortiques artificielles, dites percutanées qui s'inspire des techniques de traitement endovasculaire par introduction d'un cathéter à l'intérieur d'un vaisseau sanguin, tel que l'aorte.

Ainsi, la valve aortique cardiaque native qui est déficiente par calcification est remplacée par une valve artificielle en évitant l'intervention habituelle de chirurgie cardiaque lourde comme évoqué ci-avant.

La mise en place d'une valve artificielle peut être actuellement réalisée par différentes voies percutanées : par voie trans-fémorale, c'est-à-dire par introduction depuis l'artère fémorale jusqu'au cœur ou par voie trans-apicale, ou par voie trans-aortique, ou par voie carotidienne ou encore par voie sous-clavière, c'est-à-dire toute voie ne nécessitant ni opération à cœur ouvert par l'intermédiaire d'une ouverture du thorax ni circulation extracorporelle.

L'opération en elle-même consiste à déposer une valve artificielle (prothèse), qui reproduit la forme générale d'une valve aortique native normale, au niveau de la valve aortique native calcifiée (malade), cette dernière étant laissée en place et écrasée par la prothèse.

Pour ce faire, la valve artificielle faite en péricarde, membrane fine entourant le cœur, d'origine porcine ou bovine, est fixée préalablement à l'intérieur d'un grillage métallique tubulaire extensible radialement (« stent » en anglais) et constitué par assemblage de fils en matériau à mémoire de forme, par exemple en alliage nickel-titane ou en cobalt-chrome, acier inoxydable 316L pour les stents coronaires.

Puis l'ensemble valve-grillage est comprimé à l'extrémité d'une gaine tubulaire, appelé cathéter de délivrance, qui peut être introduit soit directement dans une artère, soit à l'intérieur d'un introducteur permettant d'accéder à l'artère tout en maintenant une hémostase.

Un médecin interventionniste fait alors coulisser l'ensemble valve-grillage dans l'introducteur ou directement dans le cathéter de délivrance, jusqu'à ce que ledit ensemble parvienne à la valve aortique malade. L'ensemble valve-grillage est ensuite déposé au niveau de la valve malade par dilatation d'un ballonnet avant la mise en place.

Il existe aussi des cathéters de délivrance de valve comprenant un ensemble valve-grillage sans ballonnet dans lesquels la valve est auto-expansible qui permet un dépôt de la valve qui s'expanse radialement par simple retrait de la gaine qui l'entoure et donc sans avoir à dilater un ballonnet au préalable.

On pourra se reporter pour plus de précisions aux brevets américains US 7018406, US 7892281, US8652202 et US 8747459.

Lors de la pose à proprement parler il est nécessaire d'effectuer pendant une courte période, un arrêt du cœur temporaire par stimulation ventriculaire rapide pour minimiser le flux transvalvulaire, i.e. entre valvules, et pour éviter à tout le moins réduire l'embolisation potentielle.

Cet arrêt du cœur temporaire aussi appelé couramment « sidération cardiaque » consiste ainsi à faire battre le cœur volontairement à 150 à 200 battements par minute de sorte qu'il n'y ait plus de contraction efficace, ce qui provoque une chute des pressions et simule une tachycardie ou fibrillation ventriculaire et donc une stabilisation du cœur.

Cette stabilisation du cœur permet la stabilisation du ballon et donc d'augmenter la précision de la mise en place de la valve artificielle en quelques secondes.

Il existe des cathéters de stimulation bipolaire, à deux électrodes, dits sondes d'entrainement ou de stimulation électro-systolique, pour la stimulation endocardiaque temporaire du ventricule droit.

Ces sondes de stimulation électro-systolique présentent un certain nombre d'inconvénients détaillés comme suit.

Tout d'abord, une telle sonde constitue un abord veineux central avec un risque de complication vasculaire surajouté pour la population de patients visée qui est fragile. Le registre français désigne « France 2 » qui répertorie les interventions de remplacement de valve aortique, couramment désignées sous l'acronyme anglais TAVI pour «Transcatheter Aortic Valve Implantation », a indiqué comme résultat un taux de risque de complications vasculaires importantes égal à 4,7%. Ce résultat est reporté en page 1709 de la publication

[1]. Ensuite, cette sonde est relativement rigide, et de ce fait sa mise en place dans le ventricule droit qui est fragile et dont la paroi est plus fine que celle du ventricule gauche, induit un risque conséquent du phénomène bien connu des médecins interventionnistes sous le terme « tamponnade », qui traduit une insuffisance circulatoire importante pouvant aller jusqu'à la mortalité du patient.

Il est à noter d'ailleurs que ce risque existe aussi bien pendant l'intervention, c'est-à-dire lors de la mise en place de la sonde électro-systolique mais aussi en postopératoire du fait de la mobilisation des patients dans leur lit et donc de la sonde encore présente qui peut alors transpercer la paroi du ventricule droit.

De plus, il y a un risque de déplacement de la sonde de stimulation électro-systolique pendant le moment crucial de la mise en place de la valve. En effet, une sonde de stimulation n'est pas fixée dans une paroi du cœur et peut donc se déplacer et ainsi engendrer une perte de capture du signal électrique de stimulation.

Le cœur n'est alors plus stimulé et donc a des mouvements importants qui gênent le placement de la valve ou du ballon.

Un autre risque lié à l'utilisation de telles sondes est le risque d'infection au site de ponction. Le registre France 2 a indiqué un taux inférieur à 1% : voir publication [1].

Enfin, un médecin interventionniste considère comme non négligeable le temps opératoire supplémentaire lié à la mise en place d'une sonde de stimulation temporaire, qui est une opération pas toujours simple à réaliser.

La publication [2] met en avant les avantages de réaliser cette stimulation ventriculaire sur le ventricule gauche et non pas sur le ventricule droit et de le faire non pas au moyen d'un cathéter de stimulation spécifique par voie transveineuse, mais en mettant en oeuvre un stimulateur cardiaque externe avec le guide-fil utilisé pour les interventions de ce type.

Ainsi, la mise en oeuvre préconisée que l'on retrouve décrite dans cette publication [2] consiste à se servir du guide-fil supportant le ballon d'expansion du stent et introduit dans le ventricule gauche, en tant que partie reliée à la cathode d'un stimulateur cardiaque et d'une électrode cutanée ou aiguille plantée dans le tissu sous cutané, en tant que support de l'anode du stimulateur cardiaque.

Les publications [3] et [4] valident dans le cas d'une intervention par angioplastie coronaire sur une population porcine, l'efficacité d'une stimulation cardiaque temporaire avec une tension électrique de stimulation moindre, par la mise en oeuvre du guide-fil supportant le ballon d'expansion du stent, en tant que partie reliée à la cathode d'un stimulateur cardiaque et d'une électrode cutanée ou aiguille plantée dans le tissu sous cutané, en tant que support de l'anode du stimulateur cardiaque.

Ainsi, ces mises en œuvre préconisées ont pour avantages d'éviter d'avoir à implanter un cathéter supplémentaire dédié, d'éviter un accès supplémentaire au cœur, de réduire le temps et le coût de l'opération, mais aussi de diminuer le taux de complications liées à l'implantation du cathéter dédié, et ce tout en permettant une stimulation aussi efficace que par le biais d'une stimulation par voie transveineuse.

En outre, comparativement aux sondes de stimulation électro-systolique du ventricule droit qui induisent le risque de tamponnade comme explicité ci-avant, le guide-fil utilisé pour cette technique est très stable et vient en appui permanent contre la paroi du ventricule gauche relativement épaisse puisqu'il sert de rail pour avancer l'ensemble stent-ballon-valve au travers de la valve.

Cela étant, cette technique nécessite tout de même la mise en place d'une électrode ou d'une aiguille sous-cutanée supplémentaire qui doit être précise, la mise en place et le maintien de pinces de connexion, de type crocodile sur deux supports éloignés.

L'inventeur de la présente invention a déposé la demande de brevet WO2016/162315 A1 qui décrit et revendique l'intégration d'une électrode d'un stimulateur cardiaque directement dans la gaine d'introduction (introducteur ou cathéter de délivrance) dans l'artère d'un patient. L'invention proposée permet de rendre plus aisée et plus rapide la mise en place et la manipulation des électrodes de stimulateur cardiaque pour le(s) chirurgien(s) en charge de l'opération.

L'inconvénient de cette demande est qu'elle nécessite la réalisation d'introducteurs ou de cathéters de délivrance spécifiques.

Or, il serait intéressant de pouvoir disposer d'une ou plusieurs solutions qui permette(nt) de conserver des introducteurs ou cathéters existants, c'est-à-dire ceux ne disposant pas d'électrode intégrée en leur sein.

De manière plus générale, il serait intéressant que la ou les solutions retenue(s) s'applique non seulement aux opérations de remplacement de valve cardiaque ou aortique mais également à toutes les opérations d'implantation lors d'interventions vasculaires périphériques.

Le but de l'invention est de répondre au moins en partie à ce(s) besoin(s).

### Exposé de l'invention

Pour ce faire, l'invention a pour objet, selon une première alternative, un ensemble de remplacement d'une valve cardiaque par voie percutanée, comprenant :
- un dispositif formant un introducteur ou un cathéter de délivrance de valve comprenant au moins une gaine tubulaire d'introduction, destinée à être introduite dans une artère d'un corps humain et le cas échéant à laisser passer un dispositif d'intervention chirurgicale,
- un cathéter accessoire, destiné à être introduit dans une artère ou une veine périphérique du corps humain;
- un manchon adapté pour être emmanché autour du cathéter accessoire, le manchon étant en matériau électrique conducteur sur au moins une partie de sa périphérie extérieure de sorte que lorsque le cathéter accessoire est introduit dans l'artère ou dans la veine périphérique du corps humain, la périphérie conductrice du manchon est en contact avec le tissu sous-cutané du corps ou avec la paroi de l'artère ou de la veine, le manchon comprenant en outre une connexion électrique à une électrode d'un stimulateur cardiaque externe au corps;
- un guide-fil destiné à être introduit dans la gaine tubulaire de l'introducteur ou du cathéter de délivrance pour l'avancement de l'implant, le guide-fil comprenant une partie métallique servant en outre de connexion à l'autre électrode du stimulateur cardiaque externe.

Selon une variante de réalisation, l'électrode du stimulateur cardiaque connectée au manchon conducteur électrique emmanché autour de la gaine d'introduction du cathéter accessoire est l'anode tandis que celle connectée à la partie métallique du guide-fil introduit dans l'introducteur ou du cathéter de délivrance est la cathode.

Avantageusement, le manchon électriquement conducteur est constitué d'une pièce monobloc en matériau conducteur, tel que du carbone.

Le manchon peut être ainsi constitué d'une gaine comprenant sur sa périphérie extérieure un revêtement conducteur électrique, tel qu'un revêtement en carbone.

Selon un mode de réalisation avantageux, le manchon est élastique de sorte à pouvoir s'emmancher sur des gaines de cathéters artériels ou veineux périphériques de différents diamètres, typiquement des diamètres externes compris entre 0,2 et 2,2 mm

L'invention a pour objet selon une deuxième alternative, un ensemble de pose d'un implant cardiaque, aortique ou artériel, comprenant :
- un dispositif formant un introducteur ou un cathéter de délivrance de valve comprenant au moins une gaine tubulaire d'introduction, destinée à être introduite dans une artère d'un corps humain et le cas échéant à laisser passer un dispositif d'intervention chirurgicale,
- un cathéter accessoire, destiné à être introduit dans une artère ou veine périphérique du corps humain, le cathéter accessoire comprenant au moins une gaine tubulaire d'introduction et au moins un élément conducteur électrique dont une portion distale est apparente sur une au moins une partie de la périphérie extérieure de la gaine de sorte à être en contact avec le tissu sous-cutané du corps ou avec l'artère ou la veine périphérique et dont une portion proximale, accessible depuis l'extérieur du corps, comprend une connexion électrique de sorte à servir de connexion à une électrode d'un stimulateur cardiaque externe au corps;
- un guide-fil, destiné à être introduit dans la gaine tubulaire de l'introducteur ou du cathéter de délivrance pour l'avancement de l'implant, le guide-fil comprenant une partie métallique servant en outre de connexion à l'autre électrode du stimulateur cardiaque externe.

On précise ici qu'un « cathéter accessoire » que l'on peut désigner par cathéter de servitude est un cathéter introduit dans une artère ou veine périphérique aussi appelée indifféremment voie accessoire, ou encore voie secondaire ou voie de servitude.

Selon un mode de réalisation, l'électrode du stimulateur cardiaque connectée au cathéter accessoire est l'anode tandis que celle connectée à la partie métallique du guide-fil introduit dans l'introducteur ou du cathéter de délivrance est la cathode.

De préférence, l'élément conducteur électrique du cathéter accessoire est un fil ou une bande métallique logé(e) au moins en partie dans l'épaisseur de la gaine dont une portion distale est apparente à la périphérie extérieure de la gaine.

La section du fil ou de la bande métallique peut avantageusement être comprise entre 0,25 et 5 mm².

L'ensemble selon l'invention peut constituer un ensemble de remplacement d'une valve cardiaque par voie percutanée, le guide-fil étant adapté pour réaliser l'avancement d'une valve artificielle destinée à remplacer la valve cardiaque.

Il peut également constituer un ensemble de pose d'une endoprothèse aortique ou d'un stent carotidien, le guide-fil étant adapté pour réaliser l'avancement de l'endoprothèse ou du stent ou le guide fil étant indépendant de celui qui amène la prothèse ou le ballonnet mais qui est couplé à un autre guide fil en contact avec le cœur du patient.

Grâce au cathéter veineux périphérique selon l'invention qui intègre un élément conducteur électrique à la périphérie de sa gaine, il n'est plus nécessaire de planter une aiguille dans les tissus sous-cutanés ou une électrode cutanée pour servir de support à l'électrode, typiquement l'anode d'un stimulateur cardiaque.

Il n'est pas non plus nécessaire d'utiliser et de mettre en place une sonde électro-systolique comme selon l'état de l'art, appelée couramment sonde temporaire.

De plus, grâce à l'invention, l'intensité de stimulation nécessaire à la sidération cardiaque est plus faible que dans les solutions selon l'état de l'art, du fait d'une impédance plus faible du système vasculaire par rapport au tissu sous-cutané. Typiquement, l'intensité du courant délivré, en vue d'une sidération cardiaque, peut aller de 1 à 20 mA et la tension délivrée de 0,5 à 15 Volt.

Le(s) chirurgien(s) en charge de l'opération peu(ven)t ainsi relier l'électrode, typiquement l'anode du stimulateur cardiaque au cathéter accessoire ou au manchon conducteur emmanché autour de du cathéter accessoire facilement puis relier comme usuellement l'autre électrode, typiquement la cathode au guide-fil de l'ensemble valve-stent-ballon ou valve auto-expansible-stent.

Ainsi, l'étape de préparation à l'arrêt du cœur est plus simple et plus rapide à réaliser.

En plus, l'inventeur pense qu'un cathéter accessoire selon l'invention peut diminuer les risques de complications liées aux sondes de stimulation électro-systolique selon l'état de l'art qui sont placées dans le ventricule droit.

L'introducteur ou le cathéter de délivrance peut être introduit comme usuellement par voie transapicale ou par voie transfémorale, qui est privilégiée pour son caractère moins invasif pour des patients plus fragiles.

L'introducteur ou le cathéter de délivrance peut intégrer un système de perfusion périphérique, usuellement appelé « Flush », qui peut être mis en place pour nettoyer l'intérieur de l'introducteur ou du cathéter de tout caillot de sang susceptible d'être présent.

La valve artificielle peut être introduite et positionnée dans l'artère au moyen d'un cathéter de valve classique, lui-même introduit dans l'introducteur. La valve artificielle occupe alors une position repliée et ne fait pas obstacle à l'introduction et coulissement du cathéter de valve dans l'introducteur puis dans l'artère ou dans le cathéter de délivrance puis dans l'artère.

Puis, en position déployée, la valve artificielle vient prendre appui contre la paroi extérieure de la valve cardiaque native en lieu et place de cette dernière en l'écrasant.

Un cathéter de valve classique permet ainsi d'introduire et de positionner la valve artificielle à l'endroit adéquat, par le même geste d'opération que celui permettant d'ouvrir et d'écraser la valve native. Après ouverture et écrasement de cette dernière, le cathéter de valve est coulissé axialement dans la direction distale afin d'amener la valve artificielle au niveau adéquat dans l'ouverture de la valve native.

Le(s) chirurgien(s) intervenant sur le patient applique(nt) pendant l'ouverture et l'écrasement de la valve native puis après, une stimulation cardiaque au moyen du stimulateur cardiaque extérieur, le courant électrique circulant entre la cathode et l'anode du stimulateur, la cathode étant reliée au guide-fil de la valve artificielle et l'anode étant reliée à l'élément conducteur du cathéter veineux périphérique selon l'invention, en contact avec le tissu sous-cutané du patient ou avec la paroi interne d'une veine périphérique.

Simultanément à la stimulation ventriculaire, la valve artificielle est déployée. Le cathéter de valve est ensuite retiré.

Les mêmes étapes peuvent être réalisées pour tous les types d'interventions vasculaires périphériques.

Une méthode de pose d'un implant cardiaque, aortique ou artériel, ne faisant pas partie de l'invention revendiquée, est aussi décrite. La méthode comprend les étapes suivantes:
a/ introduction d'un introducteur et/ou d'un cathéter de délivrance par voie percutanée ou dans une artère périphérique d'un corps humain;
b/ introduction d'un guide-fil dans la gaine tubulaire de l'introducteur ou du cathéter de délivrance pour l'avancement d'un implant cardiaque, aortique ou artériel;
c/ introduction d'un cathéter accessoire dans une artère ou veine périphérique du corps humain; le cathéter accessoire comprenant au moins une gaine tubulaire d'introduction et au moins un élément conducteur électrique dont une portion distale est apparente sur une au moins une partie de la périphérie extérieure de la gaine, ou le cathéter accessoire comprenant un manchon en matériau électrique conducteur sur au moins une partie de sa périphérie;
d/ liaison entre une des deux électrodes d'un stimulateur cardiaque extérieur et le cathéter accessoire ou le manchon emmanché autor du cathéter accessoire et liaison entre l'autre des deux électrodes du stimulateur cardiaque et le guide-fil;
e/ pose de l'implant;
g/ avant, simultanément ou après l'étape, sidération cardiaque au moyen du stimulateur cardiaque extérieur.

De manière générale, l'invention concerne la mise en oeuvre d'une anode périphérique cutannée ou sous-cutanée ou endovasculaire permettant via un guide métalique positionné dans le coeur de propager le courant électrique jusqu'au coeur et ainsi de le stimuler à une fréquence variable, typiquement entre 40 et 220 battements par mn, afin de le stabiliser temporairement en le sidérant ou de l'entrainer en cas de bradycardie ou autre trouble de conduction électrique arrêtant ou ralentissant la fréquence cardiaque lors d'un infarctus, d'une intervention coronaire, valvulaire ou artériel périphérique.

L'anode endovasculaire peut se situer n'importe ou sur le système circulatoire artériel ou veineux.

La tension appliquée pour obtenir la sidération cardiaque est de préférence comprise entre 0,5 et 15 V. Le courant appliqué est avantageusement compris entre 1 et 20mA.

L'impédance caractéristique est de préférence comprise 300 et 700 Ohms.

Pour une opération de pose de valves percutanées ou des endoprothèses aortique (TEVAR) ou des angioplasties au ballon des sténoses aortiques (Coartations de l'aorte), la fréquence du cœur que l'on cherche à obtenir par la sidération cardiaque selon l'invention est avantageusement comprise entre 140 et 220 battements par minute.

Pour une application coronaire, dans le cas d'utilisation d'un outil d'angioplastie tel que le Rotablator^{®} par exemple ou dans la prise en charge des infarctus avec troubles de conductions ou pour le traitement des artères périphériques comme les artères carotides (vectrices de ralentissement cardiaques par stimulation de récepteur autour du glomus carotidien), la fréquence est avantageusement comprise entre 50 et 100 battements par minute.

En résumé, les avantages d'un ensemble selon l'invention reprennent ceux d'un ensemble selon la demande WO2016/162315 A1 que l'on peut énumérer comme suit :
- une mise en place plus simple et plus rapide d'une électrode, typiquement l'anode de stimulation ventriculaire lors de l'opération de remplacement d'une valve aortique déficiente ou de l'opération de pose de tout implant aortique ou artériel (endoprothèse aortique thoracique, stent);
- la suppression de la nécessité de planter une aiguille sous-cutanée supplémentaire en tant que support d'électrode, typiquement d'anode de stimulateur cardiaque ;
- un temps et coût moindres de l'opération de remplacement d'une valve cardiaque déficiente ou de l'opération de pose de tout implant aortique ou artériel (endoprothèse aortique thoracique, stent) ;
- une efficacité accrue de la stimulation temporaire en vue de réaliser la sidération cardiaque souhaitée du fait de la moindre impédance du système vasculaire rencontré par le courant électrique de stimulation puisque le manchon autour d'un cathéter veineux accessoire est directement en contact avec ledit système, au contraire des aiguilles selon l'état de l'art qui viennent en contact avec le tissu cutané d'un patient qui présente nécessairement une impédance plus élevée;
- une efficacité accrue de la stimulation temporaire en vue de réaliser la sidération cardiaque souhaitée du fait de la stabilité du guide rigide (diamètre de l'ordre de 1,455mm) dans le ventricule gauche et du cathéter accessoire, au lieu de l'instabilité de la sonde électro-systolique selon l'état de l'art mise en place dans le ventricule droit ;
- la possibilité de réaliser la stimulation temporaire cardiaque avec un courant électrique moins élevé du fait de l'impédance moindre du système vasculaire rencontré entre les deux électrodes du stimulateur extérieur ;
- la suppression des risques de complication liés aux sondes de stimulation temporaires selon l'état de l'art qui sont placées dans le ventricule droit ;
- l'utilisation possible de l'introducteur ou du cathéter de délivrance pour plusieurs types d'interventions TAVI différentes, telles qu'un remplacement de valve aortique, pulmonaire, tricuspide ou mitrale. En particulier, pour le remplacement d'une valve tricuspide dégénérée, seule la technique d'introduction d'une sonde de stimulation dans le ventricule droit au moyen du guide-rail (diamètre 0,89 mm) est envisageable car il n'est pas envisageable de mettre à la fois le guide-rail et une sonde électro-systolique puisque l'expansion du ballon ou de la prothèse valvulaire viendrait comprimer la sonde avec le risque inhérent d'interruption de la stimulation ou de coincer la sonde de stimulation;
- l'utilisation possible dans la population pédiatrique lors des procédures valvulaires ou cardiaques sur des cœurs plus tachycardes, plus mobiles que dans la population adulte. De plus, il s'agit d'une population chez laquelle la ponction veineuse fémorale peut être très difficile ainsi que la mise en place d'une sonde de stimulation ventriculaire droite. Enfin, les parois ventriculaires droites infantiles sont fines et fragiles majorant ainsi le risque de complication grave telle que la tamponnade. Il s'agit d'ailleurs de la population décrite dans la publication [2] ;
- l'utilisation possible pour tout type d'intervention vasculaire périphérique.

En outre, comparativement à un ensemble selon la demande WO2016/162315 A1, un ensemble selon l'invention présente les avantages supplémentaires suivants :
- pas de modification du protocole, car l'introduction du cathéter accessoire (artériel ou veineux périphérique) selon l'invention reste la même que pour les cathéters accessoires existants. Cette opération est très simple et aisée à mettre en œuvre et peut tout-à-fait être réalisée par un(e) assistant(e) ou infirmier(ère), celui-ci (celle-ci) n'ayant pas besoin d'avoir de compétences particulières pour cette tâche.
- augmentation de la sécurité d'intervention d'un TAVI ou de tout autre type d'intervention cardiaque (valve mitrale, valve triscupide) ou tout type d'intervention vasculaire périphérique, car le cathéter périphérique (accessoire) est retiré en dernier lors de la procédure. Avec le cathéter accessoire et donc la connexion au stimulateur cardiaque installée jusqu'au dernier moment, on peut relancer une stimulation cardiaque même en situation d'urgence ;
- possibilité d'utiliser n'importe quel type de cathéter de délivrance ou introducteur de TAVI ou autre type de valve du cœur ou pour ceux destinés aux interventions vasculaires périphériques existants, puisque les électrodes de stimulation reliées respectivement au guide-fil et au cathéter artériel ou veineux n'impactent absolument pas le choix du cathéter de délivrance ou introducteur utilisé dans le TAVI.

La demande décrit aussi l'utilisation de l'ensemble avec introducteur ou cathéter de délivrance et avec un cathéter veineux périphérique décrit précédemment pour le remplacement d'une valve aortique, pulmonaire, tricuspide ou mitrale ou encore la pose d'une endoprothèse aortique thoracique ou d'un stent carotidien.

### Description détaillée

D'autres avantages et caractéristiques de l'invention ressortiront mieux à la lecture de la description détaillée de l'invention faite à titre illustratif et non limitatif en référence aux figures suivantes parmi lesquelles:
- la figure 1 est une vue en perspective d'un introducteur selon l'état de l'art, destiné à être introduit dans une artère fémorale au niveau de l'aine d'un patient ;
- les figures 2A à 2C montrent en vue de coupe longitudinale partielle différentes étapes de coulissement d'un cathéter de valve dans l'introducteur selon la figure 1, afin de réaliser le placement d'une valve artificielle en lieu et place d'une valve aortique native déficient ;
- la figure 3 montre en vue schématique en perspective depuis l'extérieur d'un patient à la fois l'étape de mise en place d'un cathéter de valve et des électrodes de stimulation cardiaque selon l'état de l'art ;
- la figure 4 est une vue schématique en perspective d'un cathéter de délivrance selon l'état de l'art, destiné à être introduit directement dans l'artère d'un patient sans nécessité d'un introducteur ;
- la figure 5 illustre de manière schématique l'utilisation d'un ensemble selon l'invention avec un cathéter veineux selon l'invention et avec un cathéter de délivrance de la valve prothétique introduit directement dans une artère d'un patient.

Dans la description qui va suivre ainsi que dans l'ensemble de la présente demande, les termes « distal » et « proximal » sont utilisés par référence avec le corps d'un patient dont la valve aortique native déficiente est remplacée par une valve aortique artificielle. Ainsi, l'extrémité distale d'un introducteur est l'extrémité située le plus à l'intérieur du patient lors de l'opération de remplacement.

Par souci de simplification, les mêmes éléments dans un ensemble selon l'invention et dans celui selon l'état de l'art sont désignés par les mêmes références.

On précise que les différents éléments ne sont pas nécessairement représentés à l'échelle.

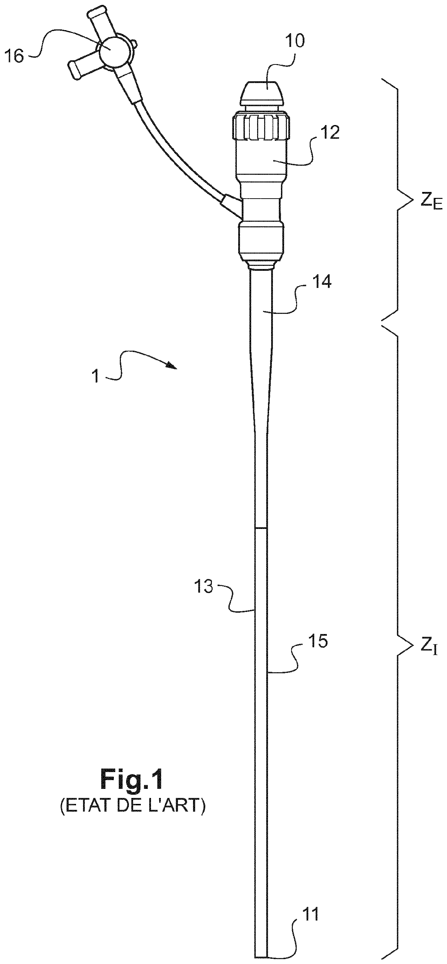

La figure 1 représente un introducteur 1 de remplacement d'une valve cardiaque par voie transfémorale.

Cet introducteur 1 de forme générale tubulaire comprend entre son extrémité proximale 10 et son extrémité distale 11, un embout 12 prolongé par au moins une gaine tubulaire 13 extérieure formée de deux portions tubulaires 14, 15 du côté proximal vers le côté distal, considérés par rapport à l'introduction dans une artère fémorale d'un patient à opérer, c'est-à-dire de haut en bas sur la figure 1.

L'embout 12 intègre en général un ensemble de vannes étanches pour réaliser une hémostase, c'est-à-dire pour assurer le maintien du sang à l'intérieur des vaisseaux sanguins du patient lors de l'intervention.

La gaine tubulaire 13 peut être extensible ou non pour pouvoir laisser passer un dispositif d'intervention chirurgicale tel qu'un cathéter de valve comme expliqué par la suite. Le matériau constitutif de la gaine 13 est un matériau biocompatible, tel qu'en silicone. Elle peut tout aussi bien être réalisée en Teflon^{™} ou en polyuréthane. La gaine peut être avantageusement recouverte à l'extérieur d'une couche hydrophile et à l'intérieur d'une couche à matériau à faible coefficient de frottement pour faciliter le coulissement d'un dispositif d'intervention.

L'introducteur 1 illustré en figure 1 comprend également un dispositif de rinçage 16 à robinets, couramment appelé « flush », intégré pour rincer l'intérieur de l'introducteur 1 au moyen d'un liquide de rinçage approprié.

Tous les éléments de l'introducteur 1 présents dans la zone proximale ou extérieure Z_{E} sont destinés à rester à l'extérieur du corps du patient, toute la portion distale 15 de la gaine 13 définissant la zone distale Z_{I} est destinée à être introduite dans une artère fémorale d'un patient.

L'introducteur 1 illustré est par exemple celui commercialisé sous la dénomination commerciale « *ensemble de gaine d'introduction eSheath d'Edwards »* commercialisé par la société Edwards Lifesciences.

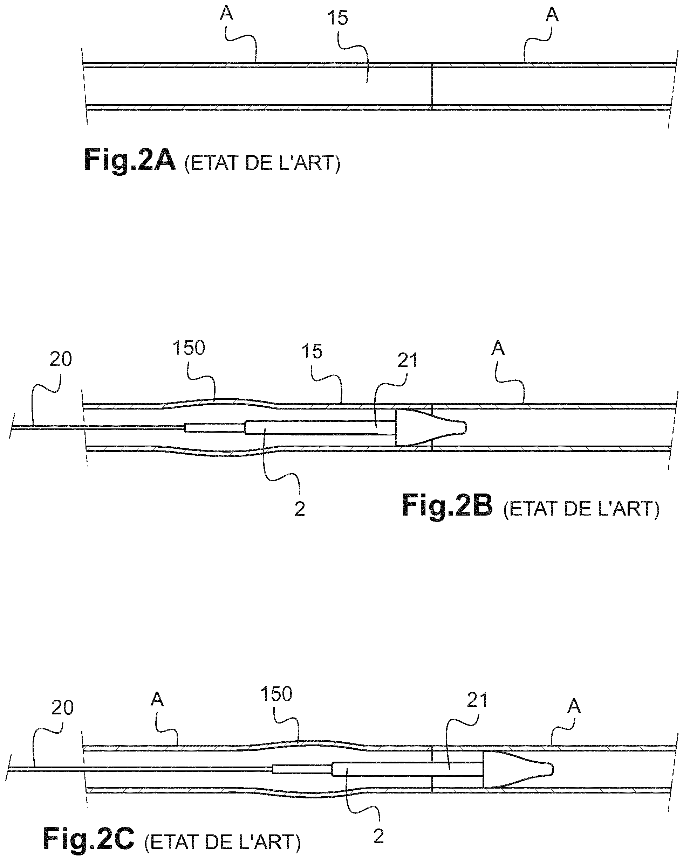

On a représenté aux figures 2A à 2C l'avancement d'un cathéter de valve 2 constitué d'un guide-fil 20 et d'un ensemble 21 constitué d'une valve artificielle fixée à un stent à expansion radiale et d'un ballon gonflable pour réaliser cette expansion, à l'intérieur de la portion distale 14 de la gaine tubulaire de l'introducteur 1 déjà introduit dans une artère fémorale A.

La pointe de l'ensemble 21 permet de pénétrer aisément la valve aortique native déficiente.

On voit sur ces figures qu'au fur et à mesure du coulissement du cathéter de valve 2, la portion 15 de la gaine tubulaire se déforme temporairement radialement en formant une légère excroissance 150. Lorsque la gaine tubulaire n'est pas extensible, elle ne se déforme pas radialement.

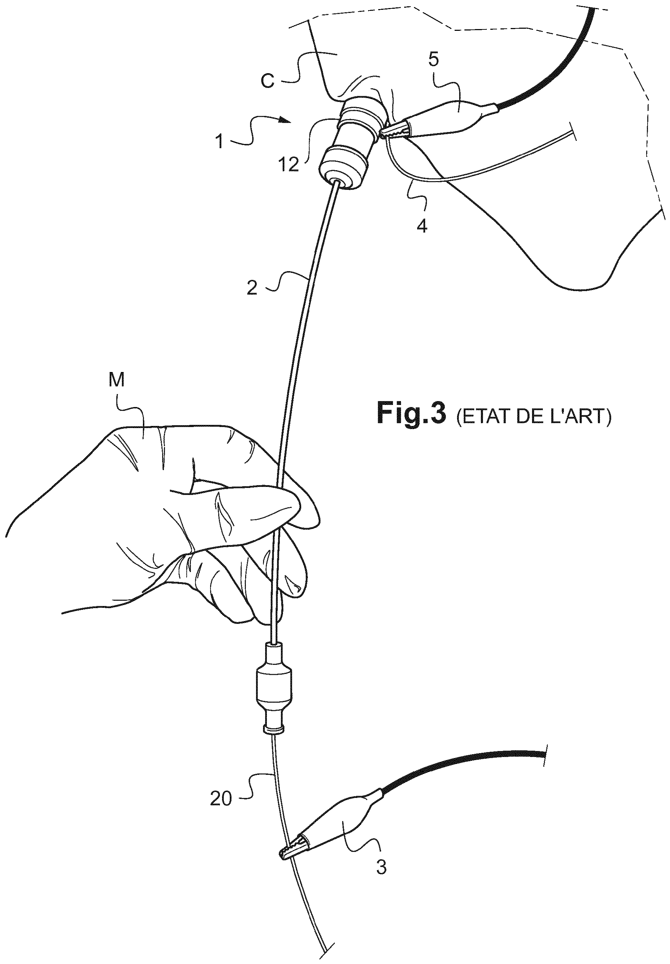

En figure 3, on peut voir que la main M d'un chirurgien réalise l'introduction du cathéter de valve 2 dans l'introducteur 1 déjà introduit dans l'artère fémorale d'un patient, l'embout 12 faisant saillie à l'extérieur du corps C.

Cette introduction du cathéter de valve 2 permet d'amener l'ensemble 21 au niveau de la valve aortique calcifiée déficiente qu'il faut remplacer.

Usuellement, comme visible également sur cette figure 3, une pince 3 connue sous l'appellation pince-crocodile est fixée par pincement sur le guide-fil 20 du cathéter de valve 2. Cette pince 3 est reliée à la cathode d'un stimulateur cardiaque non représenté, situé à l'extérieur du corps C.

Une aiguille non montrée est également plantée dans les tissus sous-cutanés du corps C du patient à opérer. Sur cette aiguille est fixé un fil métallique 4.

Une pince 5 de type crocodile est fixée également par pincement sur le fil métallique 4.

Cette pince 5 est reliée à l'anode du stimulateur cardiaque extérieur au corps.

Ainsi, lorsque la valve artificielle est au niveau de la valve aortique native à remplacer, le chirurgien réalise préalablement à la mise en place proprement dite de la valve artificielle, c'est-à-dire au gonflement du ballon et donc l'expansion du stent auquel est fixée la valve, une stimulation ventriculaire rapide du ventricule gauche.

Pour cela, un signal électrique est délivré entre la cathode et l'anode par le biais des pinces 3, 5, le ballon jouant le rôle d'isolant électrique entre ces deux électrodes.

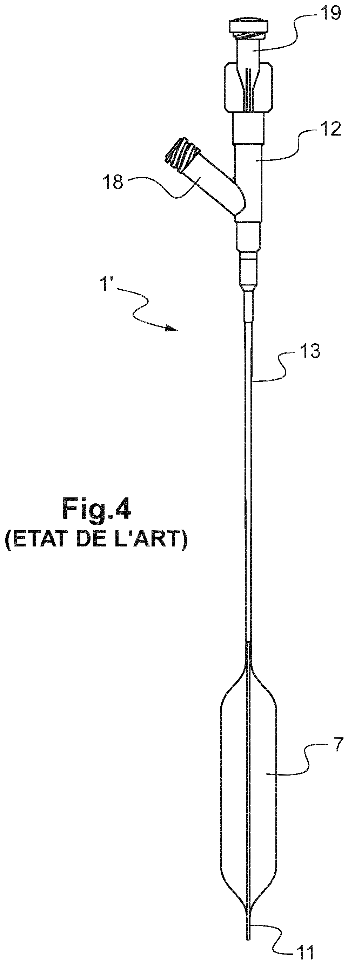

La figure 4 illustre un cathéter de délivrance 1' qui peut être introduit directement dans l'artère d'un patient sans nécessité d'un introducteur. Plus précisément, le cathéter 1' comprend un embout 12 prolongé par une gaine d'introduction 13. L'embout 12 comprend une connexion 18 pour le gonflage/dégonflage d'un ballonnet 7 à l'extrémité distale 11 qui permet d'expandre une valve prothétique non représentée.

Confronté à de nombreuses opérations de remplacement de valve aortique par voie fémorale telle qu'elle vient d'être brièvement décrite, et en particulier à la mise en place précise et délicate de l'aiguille sous-cutanée supplémentaire, ainsi que la mise en place et le maintien des pinces de connexion, de type crocodile sur deux supports éloignés, l'inventeur de la présente invention a déjà pensé à intégrer le fil métallique 4 directement dans un introducteur 1 ou dans un cathéter de délivrance 1'. Cette solution est décrite et revendiquée dans la demande de brevet WO2016/162315 A1.

Si cette solution amène de nombreux avantages par rapport à la technique selon l'état de l'art, elle présente néanmoins un inconvénient important qui est de nécessiter la réalisation d'introducteurs ou cathéters de délivrance spécifiques.

Aussi, l'inventeur a tout d'abord pensé à intégrer la fonction du fil métallique non pas dans un introducteur ou dans un cathéter de délivrance spécifique mais à l'intégrer dans un cathéter veineux périphérique 1" existant.

Un tel cathéter veineux 1" qui peut être de faible diamètre, typiquement de 2mm ou moins, et de faible longueur. Il est conforme aux normes relatives aux cathéters intravasculaire périphériques. On rappelle ici que la fonction première d'un cathéter veineux périphérique est d'amener un liquide de réhydratation du patient, un traitement médicamenteux ou une transfusion.

Plus particulièrement, dans le cadre d'un TAVI, le cathéter artériel 1" permet de pouvoir réaliser une angiographie de contrôle, d'une part afin de contrôler le positionnement de la valve aortique prothétique et d'autre part, afin de vérifier l'absence de complications, notamment vasculaires voire de les gérer. ( La fonction première d'un cathéter veineux périphérique est d'amener un liquide de réhydratation du patient, un traitement médicamenteux ou une transfusion. )

Selon l'invention, la gaine d'introduction d'un cathéter artériel périphérique 1" intègre en périphérie un fil ou une bande électriquement conductrice, de très faible épaisseur, typiquement de l'ordre du millimètre ou inférieure, qui ne rajoute qu'une faible surépaisseur à une gaine classique de cathéter veineux et donc ne gêne pas la progression celle-ci lors de son introduction dans une artère ou veine périphérique V.

En pratique, un chirurgien ou médecin interventionniste souhaitant procéder à une opération de remplacement de valve stimulation cardiaque avec stimulation cardiaque concomitante à la mise en place de la valve prothétique, commence par mettre en place le cathéter artériel ou veineux périphérique 1" de sorte que l'élément conducteur de sa gaine vienne toucher soit une zone sous cutanée du patient, soit la paroi de l'artère ou de la veine périphérique du patient.

Une fois cette mise en place effectuée, l'introduction de l'introducteur 1 ou du cathéter de délivrance 1', peut être faite comme usuellement par le chirurgien.

Une fois le positionnement de l'introducteur 1 ou du cathéter 1' dans l'artère fémorale A achevé, la connexion électrique 6 peut être connectée directement à l'anode d'un stimulateur cardiaque externe 9 par le biais d'un fil de connexion 90.

Usuellement, une pince, comme la pince-crocodile 3 représentée en figure 3 peut à son tour être est fixée par pincement sur le guide-fil 20 d'un introducteur 1 ou d'un cathéter de valve 1'. Cette pince est reliée à la cathode du stimulateur cardiaque externe par le biais d'un fil de connexion 91.

Ainsi, la stimulation cardiaque temporaire pour réaliser la sidération cardiaque souhaitée, peut avoir lieu entre la cathode reliée électriquement au guide-fil 20 et l'anode reliée électriquement à l'élément conducteur électrique du cathéter veineux périphérique 1".

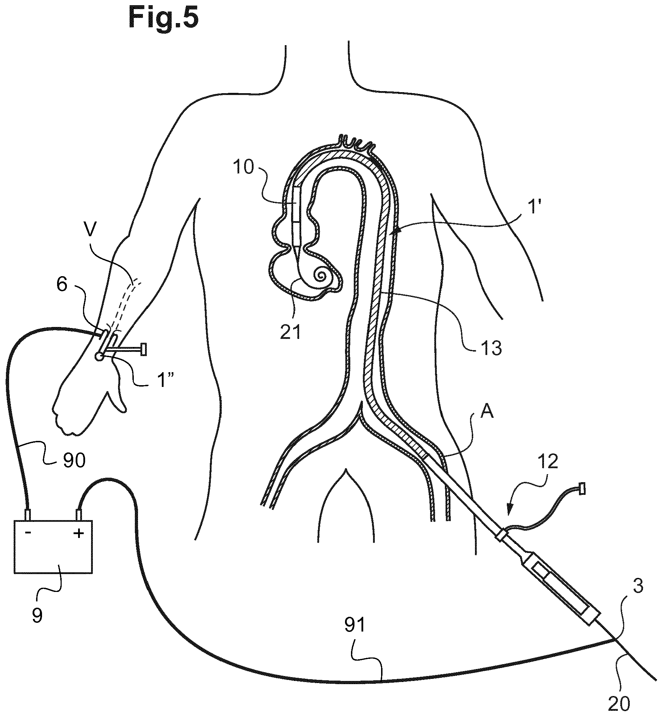

La figure 5 schématise l'ensemble des éléments mis enjeu, à savoir l'utilisation d'un ensemble avec cathéter de délivrance 1' pour la délivrance d'une valve prothétique 10 par l'arche aortique d'un patient et avec un cathéter veineux périphérique 1" selon l'invention.

Comme on peut le voir sur cette figure 5, le cathéter 1' est introduit depuis l'artère fémorale et un guide-fil 20 est introduit directement dans le cathéter de délivrance 1'. L'extrémité distale 21 du guide-fil 20 s'enroule sur elle-même en venant en contact avec l'endothélium ventriculaire gauche du patient. Cette extrémité distale 21 du guide-fil 20 assure toujours le contact électrique et donc le passage du courant depuis la cathode du stimulateur cardiaque externe 9 via le fil électrique de connexion 91.

Le passage du courant vers l'anode du stimulateur cardiaque 9 est quant à lui assuré par la gaine conductrice du cathéter veineux périphérique 1" et donc en contact avec le tissu sous-cutané du patient ou la veine périphérique et relié par ailleurs au fil électrique de connexion 90.

L'invention n'est pas limitée aux exemples qui viennent d'être décrits ; on peut notamment combiner entre elles des caractéristiques des exemples illustrés au sein de variantes non illustrées.

D'autres variantes et améliorations peuvent être prévues sans pour autant sortir du cadre de l'invention qui est défini par les revendications.

### Références citées

[1]: « Registry of Transcatheter Aortic-Valve Implantation in High-Risk Patients », Gilard et al ; the New England Journal of Medicine : pp 1705-1715
[2] : « Left Ventricular Guidewire Pacing to Simplify Aortic Balloon Valvuloplasty », Susanne Navarini et al; Catheterization and Cardiovascular Interventions 73: pp 426-427 (2009)
[3] : « A novel Approach for Transcoronary Pacing un a Porcine Model », Roland Prodzinsky et al ; Journal of Invasive Cardiology 24(9) : pp 451-455 (2012)
[4] : « Optimizing of Transcoronary Pacing in a Porcine Model », Konstantin M. Heinroth, et al, Journal of Invasive Cardiology 21, pp 634-638 (2009)

## Revendications

1. Ensemble de pose d'un implant cardiaque, aortique ou artériel, comprenant
- un dispositif formant un introducteur (1) ou un cathéter de délivrance de valve (1') comprenant au moins une gaine tubulaire (13) d'introduction, destinée à être introduite dans une artère d'un corps humain (C) et le cas échéant à laisser passer un dispositif d'intervention chirurgicale,
- un cathéter accessoire (1"), destiné à être introduit dans une artère ou une veine périphérique du corps humain;
- un manchon (6) adapté pour être emmanché autour du cathéter accessoire, le manchon étant en matériau électrique conducteur sur au moins une partie de sa périphérie extérieure de sorte que lorsque le cathéter accessoire est introduit dans l'artère ou dans la veine périphérique du corps humain, la périphérie conductrice du manchon est en contact avec le tissu sous-cutané du corps ou avec la paroi de l'artère ou de la veine, le manchon comprenant en outre une connexion électrique (7, 8) à une électrode d'un stimulateur cardiaque externe au corps;
- un guide-fil (20) destiné à être introduit dans la gaine tubulaire (13) de l'introducteur ou du cathéter de délivrance pour l'avancement de l'implant, le guide-fil (20) comprenant une partie métallique servant en outre de connexion à l'autre électrode du stimulateur cardiaque externe.

2. Ensemble selon la revendication 1, l'électrode du stimulateur cardiaque connectée au manchon conducteur électrique (6) emmanché autour de la gaine d'introduction (14") du cathéter accessoire étant l'anode tandis que celle connectée à la partie métallique du guide-fil (20) introduit dans l'introducteur (1) ou du cathéter de délivrance est la cathode.

3. Ensemble selon l'une des revendications 1 ou 2, dans lequel le manchon électriquement conducteur est constitué d'une pièce monobloc en matériau conducteur, tel que du carbone.

4. Ensemble selon revendication 3, le manchon étant constitué d'une gaine comprenant sur sa périphérie extérieure un revêtement conducteur électrique (4), tel qu'un revêtement en carbone.

5. Ensemble selon la revendication 3 ou 4, le manchon étant élastique de sorte à pouvoir s'emmancher sur des gaines de cathéters artériels ou veineux périphériques de différents diamètres, typiquement des diamètres externes compris entre 0,2 et 2,2 mm.

6. Ensemble de pose d'un implant cardiaque, aortique ou artériel, comprenant :
- un dispositif formant un introducteur (1) ou un cathéter de délivrance de valve (1') comprenant au moins une gaine tubulaire (13) d'introduction, destinée à être introduite dans une artère d'un corps humain (C) et le cas échéant à laisser passer un dispositif d'intervention chirurgicale,
- un cathéter accessoire (1"), destiné à être introduit dans une artère ou une veine périphérique du corps humain, le cathéter accessoire comprenant au moins une gaine tubulaire (13) d'introduction et au moins un élément conducteur électrique dont une portion distale est apparente sur une au moins une partie de la périphérie extérieure de la gaine de sorte à être en contact avec le tissu sous-cutané du corps ou avec l'artère ou la veine périphérique et dont une portion proximale, accessible depuis l'extérieur du corps (C), comprend une connexion électrique de sorte à servir de connexion à une électrode d'un stimulateur cardiaque externe au corps;
- un guide-fil (20) destiné à être introduit dans la gaine tubulaire (13) de l'introducteur ou du cathéter de délivrance pour l'avancement de l'implant, le guide-fil (20) comprenant une partie métallique servant en outre de connexion à l'autre électrode du stimulateur cardiaque externe.

7. Ensemble selon la revendication 6, l'électrode du stimulateur cardiaque connectée au cathéter accessoire étant l'anode tandis que celle connectée à la partie métallique du guide-fil (20) introduit dans l'introducteur (1) ou du cathéter de délivrance est la cathode.

8. Ensemble selon l'une des revendications 6 ou 7, dans lequel l'élément conducteur électrique du cathéter accessoire est un fil ou une bande métallique (4) logé(e) au moins en partie dans l'épaisseur de la gaine dont une portion distale est apparente à la périphérie extérieure de la gaine.

9. Ensemble selon la revendication 8, la section du fil ou de la bande métallique étant comprise entre 0,25 et 5 mm².

10. Ensemble selon l'une des revendications précédentes, constituant un ensemble de remplacement d'une valve cardiaque par voie percutanée, le guide-fil étant adapté pour réaliser l'avancement d'une valve artificielle destinée à remplacer la valve cardiaque.

11. Ensemble selon l'une des revendications précédentes, constituant un ensemble de pose d'une endoprothèse aortique ou d'un stent carotidien, le guide-fil étant adapté pour réaliser l'avancement de l'endoprothèse ou du stent ou le guide fil étant indépendant de celui qui amène la prothèse ou le ballonnet mais qui est couplé à un autre guide fil en contact avec le cœur du patient.

## Patentansprüche

1. Anordnung zur Einsetzung eines Herz-, Aorten- oder Arterienimplantats, umfassend:
- eine Vorrichtung, die einen Inserter (1) oder einen Katheter zur Einbringung einer Klappe (1') bildet, der mindestens eine röhrenförmige Einführhülle (13) umfasst, die dazu bestimmt ist, in eine Arterie eines menschlichen Körpers (C) eingeführt zu sein und gegebenenfalls eine Vorrichtung für einen chirurgischen Eingriff hindurchzulassen,
- einen Hilfskatheter (1"), der dazu bestimmt ist, in eine periphere Arterie oder Vene des menschlichen Körpers eingeführt zu sein;
- eine Muffe (6), die geeignet ist, um den Hilfskatheter herum anzuliegen, wobei die Muffe auf wenigstens einem Abschnitt ihres Außenumfangs aus elektrisch leitendem Material ist, so dass, wenn der Hilfskatheter in die periphere Arterie oder Vene des menschlichen Körpers eingeführt ist, der leitende Umfang der Muffe mit dem Subkutangewebe des Körpers oder mit der Wand der Arterie oder Vene in Kontakt steht, wobei die Muffe ferner einen elektrischen Anschluss (7, 8) an eine Elektrode eines Herzschrittmachers außerhalb des Körpers umfasst;
- eine Drahtführung (20) zur Einführung in die röhrenförmige Hülle (13) des Inserters oder des Einbringungskatheters zur Voranbewegung des Implantats, wobei die Drahtführung (20) einen metallischen Abschnitt umfasst, der ferner als Anschluss an die andere Elektrode des äußeren Herzschrittmachers dient.

2. Anordnung nach Anspruch 1, wobei die Elektrode des Herzschrittmachers, die mit der elektrisch leitenden Muffe (6) verbunden ist, die um die Einführhülle (14") des Hilfskatheters herum anliegt, die Anode ist, während diejenige, die mit dem metallischen Abschnitt der Drahtführung (20), die in den Inserter (1) eingeführt ist, oder des Einbringungskatheter verbunden ist, die Kathode ist.

3. Anordnung nach einem der Ansprüche 1 oder 2, wobei die elektrisch leitende Muffe von einem einstückigen Teil aus leitendem Material wie etwa Kohlenstoff gebildet ist.

4. Anordnung nach Anspruch 3, wobei die Muffe von einer Hülle gebildet ist, die an ihrem Außenumfang eine elektrisch leitende Beschichtung (4) umfasst, wie etwa eine Kohlenstoffbeschichtung.

5. Anordnung nach Anspruch 3 oder 4, wobei die Muffe elastisch ist, so dass sie an Hüllen von peripheren Arterien- oder Venenkathetern mit verschiedenen Durchmessern, typischerweise Außendurchmessern zwischen 0,2 und 2,2 mm, anliegen kann.

6. Anordnung zur Einsetzung eines Herz-, Aorten- oder Arterienimplantats, umfassend:
- eine Vorrichtung, die einen Inserter (1) oder einen Katheter zur Einbringung einer Klappe (1') bildet, der mindestens eine röhrenförmige Einführhülle (13) umfasst, die dazu bestimmt ist, in eine Arterie eines menschlichen Körpers (C) eingeführt zu sein und gegebenenfalls eine Vorrichtung für einen chirurgischen Eingriff hindurchzulassen,
- einen Hilfskatheter (1"), der dazu bestimmt ist, in eine periphere Arterie oder Vene des menschlichen Körpers eingeführt zu sein, wobei der Hilfskatheter wenigstens eine röhrenförmige Einführhülle (13) und wenigstens ein elektrisch leitendes Element umfasst, dessen ein distaler Abschnitt auf wenigstens einem Abschnitt des Außenumfangs der Hülle sichtbar ist, so dass er mit dem Subkutangewebe des Körpers oder mit der peripheren Arterie oder Vene in Kontakt steht, und dessen ein proximaler Abschnitt, der von außerhalb des Körpers (C) zugänglich ist, einen elektrischen Anschluss umfasst, um als Anschluss an eine Elektrode eines Herzschrittmachers außerhalb des Körpers zu dienen;
- eine Drahtführung (20) zur Einführung in die röhrenförmige Hülle (13) des Inserters oder des Einbringungskatheters zur Voranbewegung des Implantats, wobei die Drahtführung (20) einen metallischen Abschnitt umfasst, der ferner als Anschluss an die andere Elektrode des äußeren Herzschrittmachers dient.

7. Anschluss nach Anspruch 6, wobei die Elektrode des Herzschrittmachers, die an den Hilfskatheter angeschlossen ist, die Anode ist, während diejenige, die mit dem metallischen Abschnitt der Drahtführung (20), die in den Inserter (1) eingeführt ist, oder des Einbringungskatheters verbunden ist, die Kathode ist.

8. Anordnung nach einem der Ansprüche 6 oder 7, wobei das elektrisch leitende Element des Hilfskatheters ein Metalldraht oder -band (4) ist, das wenigstens teilweise in der Dicke der Hülle aufgenommen ist, dessen ein distaler Abschnitt am Außenumfang der Hülle sichtbar ist.

9. Anordnung nach Anspruch 8, wobei der Querschnitt des Metalldraht oder -bands zwischen 0,25 und 5 mm² beträgt.

10. Anordnung nach einem der vorhergehenden Ansprüche, die eine Anordnung zur perkutanen Ersetzung einer Herzklappe bildet, wobei die Drahtführung geeignet ist, die Voranbewegung einer künstlichen Klappe zu erreichen, die dazu bestimmt ist, die Herzklappe zu ersetzen.

11. Anordnung nach einem der vorhergehenden Ansprüche, die eine Anordnung zur Einsetzung einer Aortenendoprothese oder eines Karotis-Stents bildet, wobei die Drahtführung geeignet ist, die Voranbewegung der Endoprothese oder des Stents auszuführen, oder die Drahtführung von derjenigen unabhängig ist, die die Prothese oder den Ballon einbringt, die jedoch an eine andere Drahtführung gekoppelt ist, die mit dem Herzen des Patienten in Kontakt steht.

## Claims

1. Assembly for placement of a cardiac, aortic or arterial implant, comprising:
- a device forming an introducer (1) or a valve delivery catheter (1') comprising at least one tubular insertion sheath (13), intended to be introduced into an artery of a human body (C) and optionally to permit the passage of a surgical intervention device;
- an accessory catheter (1") intended to be introduced into a peripheral vein or artery of the human body;
- a sleeve (6) adapted to be engaged around the accessory catheter, the sleeve being made of electrically conductive material over at least part of its outer periphery, such that, when the accessory catheter is introduced into the peripheral vein or artery of the human body, the conductive periphery of the sleeve is in contact with the subcutaneous tissue of the body or with the wall of the artery or of the vein, the sleeve additionally comprising an electrical connection (7, 8) to an electrode of a cardiac stimulator outside the body;
- a guidewire (20) intended to be introduced into the tubular sheath (13) of the introducer or of the delivery catheter for advancing the implant, the guidewire (20) comprising a metal part additionally serving as a connection to the other electrode of the external cardiac stimulator.

2. Assembly according to Claim 1, the electrode of the cardiac stimulator connected to the electrically conductive sleeve (6) engaged around the insertion sheath (14") of the accessory catheter being the anode, while the one connected to the metal part of the guidewire (20) introduced into the introducer (1) or the delivery catheter is the cathode.

3. Assembly according to either of Claims 1 and 2, in which the electrically conductive sleeve is formed as one piece made of conductive material, for example carbon.

4. Assembly according to Claim 3, the sleeve being formed by a sheath comprising on its outer periphery an electrically conductive coating (4), for example a coating of carbon.

5. Assembly according to Claim 3 or 4, the sleeve being elastic so as to be able to engage on peripheral venous or arterial catheter sheaths of different diameters, typically external diameters of between 0.2 and 2.2 mm.

6. Assembly for placement of a cardiac, aortic or arterial implant, comprising:
- a device forming an introducer (1) or a valve delivery catheter (1') comprising at least one tubular insertion sheath (13), intended to be introduced into an artery of a human body (C) and optionally to permit the passage of a surgical intervention device;
- an accessory catheter (1"), intended to be introduced into a peripheral vein or artery of the human body, the accessory catheter comprising at least one tubular insertion sheath (13) and at least one electrically conductive element, of which a distal portion is exposed on at least one part of the outer periphery of the sheath in such a way as to be in contact with the subcutaneous tissue of the body or with the peripheral vein or artery, and of which a proximal portion, accessible from the outside of the body (C), comprises an electrical connection so as to serve as a connection to an electrode of a cardiac stimulator outside the body;
- a guidewire (20) intended to be introduced into the tubular sheath (13) of the introducer or of the delivery catheter for advancing the implant, the guidewire (20) comprising a metal part additionally serving as a connection to the other electrode of the external cardiac stimulator.

7. Assembly according to Claim 6, the electrode of the cardiac stimulator connected to the accessory catheter being the anode, while the one connected to the metal part of the guidewire (20) inserted into the introducer (1) or the delivery catheter is the cathode.

8. Assembly according to either of Claims 6 and 7, in which the electrically conductive element of the accessory catheter is a wire or a metal band (4) housed at least partially within the thickness of the sheath, of which a distal portion is exposed at the outer periphery of the sheath.

9. Assembly according to Claim 8, the cross section of the wire or of the metal band being between 0.25 and 5 mm².

10. Assembly according to one of the preceding claims, constituting an assembly for replacement of a cardiac valve by a percutaneous route, the guidewire being adapted for the advance of an artificial valve intended to replace the cardiac valve.

11. Assembly according to one of the preceding claims, constituting an assembly for placement of an aortic endoprosthesis or carotid stent, the guidewire being adapted for the advance of the endoprosthesis or stent, or the guidewire being independent of the one which delivers the prosthesis or the balloon but which is coupled to another guidewire in contact with the patient's heart.
